Europäisches Patentamt

European Patent Office

Office européen des brevets

(11) Numéro de publication: **0 468 121 A1**

## DEMANDE DE BREVET EUROPEEN

(21) Numéro de dépôt: 90402150.8

(22) Date de dépôt: 26.07.90

(51) Int. Cl.5: **A61K 9/18**, A61K 9/20, A61K 31/60

(43) Date de publication de la demande:
**29.01.92 Bulletin 92/05**

(84) Etats contractants désignés:
**AT BE CH DE DK ES FR GB GR IT LI LU NL SE**

(71) Demandeur: **Perovitch, Philippe**
**251, avenue de la Marne**
**F-33700 Mérignac(FR)**

Demandeur: **Maury, Marc**
**17, rue des Augustins**
**F-33000 Bordeaux(FR)**

(72) Inventeur: **Perovitch, Philippe**
**251, avenue de la Marne**
**F-33700 Mérignac(FR)**
Inventeur: **Maury, Marc**
**17, rue des Augustins**
**F-33000 Bordeaux(FR)**

(74) Mandataire: **Wagret, Jean-Michel**
**23 rue de Léningrad**
**F-75008 Paris(FR)**

(54) **Procédé de préparation galénique d'une composition thérapeutique notamment à base d'aspirine.**

(57) Procédé de préparation d'une composition thérapeutique à usage interne, notamment à administration per os, sous présentation galénique, et contenant au moins un principe actif sous forme solide adsorbé sur un substrat, le produit actif y étant réparti à l'état de microparticules finement dispersées et le procédé étant du type comportant les phases :
- de mise en solution du principe actif de départ dans un solvant approprié,
- d'imprégnation de la solution ainsi formée sur un support alimentaire de structure appropriée et pharmaceutiquement compatible avec ledit principe actif,
- d'évaporation du solvant de la solution adsorbée au sein dudit support en provoquant la reconstitution du principe actif par la formation de microcristaux adsorbés sur les parois et au sein du substrat,
- et le procédé est caractérisé en ce que le solvant est constitué d'un système binaire comportant un solvant principal, de nature organique, et un adjuvant, de type solide à la température ambiante et soluble dans le solvant principal, l'adjuvant présentant par ailleurs des propriétés lubrifiantes

EP 0 468 121 A1

L'invention concerne la présentation galénique d'une composition thérapeutique.

On a cherché à obtenir des produits actifs à l'état microdispersé et répartis à l'intérieur d'un substrat lui-même de structure réceptrice en procédant à la mise en solution du principe actif (insoluble ou très peu soluble dans l'eau) dans un solvant organique, après quoi le substrat ainsi imprégné de la solution est alors soumis à une phase d'évaporation du solvant, le produit actif étant ainsi contraint à recristalliser à l'intérieur de la structure support sous forme de cristaux de faibles dimensions et présentant par conséquent la surface spécifique importante recherchée.

Un procédé de ce type est notamment décrit dans la demande de brevet européen 0287488, mais malgré l'intérêt théorique de ce procédé il est apparu que sa mise en oeuvre rencontrait des difficultés pratiques au niveau de l'exploitation de la fabrication, dans un cadre industriel.

La principale difficulté résulte du manque d'homogénéité entre le substrat et les particules constitutives des microcristaux adsorbés au sein et à la surface du substrat notamment à la surface des anfractuosités de ce dernier.

A défaut d'une liaison homogène entre les deux solides, leur cohésion ne peut être assurée lors des phases que doivent successivement subir le substrat et son contenu pour être acheminés jusqu'à la forme galénique définitive acceptable, celle d'un comprimé ou d'une tablette.

On constate en effet que lors de la phase d'évaporation sous vide, par exemple, la force d'aspiration qu'il est nécessaire de mettre en oeuvre pour assurer un traitement rentable et une évacuation rapide du solvant, aboutit à un démélange de l'ensemble, les microcristaux constitués, comme recherchés, d'une poussière infinitésimale, de l'ordre de quelques microns, sont entraînés et en quelque sorte élués par le courant gazeux qui les aspire et qui appauvrit par conséquent le substrat en entraînant par ailleurs une perte notable en produits actifs.

Ce phénomène se retrouve lors des phases ultérieures de traitement, la différence de taille entre le substrat et les microcristaux entraîne des problèmes difficiles lors de la mise en comprimés. La présence des microcristaux constitue un surcroît de forces de frottement et d'adhérence et limite en effet les propriétés d'écoulement du mélange dans les presses et la mise en oeuvre de machines de production à cadences élevées et à hauts rendements est plus que compromise.

Enfin, la surface spécifique élevée des microcristaux, qui est souhaitable dans le cadre du but recherché par l'invention, augmente les phénomènes de grippage rencontrés lors de la fabrication des comprimés, les lubrifiants utilisés classiquement pour la fabrication des comprimés n'étant pas suffisamment efficaces pour assurer le maintien des conditions de cadences et de qualité souhaitables, en particulier lorsque les phénomènes de grippage devenant trop importants provoquent des défauts de qualité des comprimés.

De sorte que la mise en oeuvre d'une technique de microdispersion sur substrat n'a pu être pratiquement obtenue industriellement malgré l'intérêt qu'elle présenterait sur le plan galénique et commercial.

L'invention permet de remédier à cet inconvénient et elle prévoit des conditions qui permettent d'assurer l'homogénéité du substrat et des microcristaux adsorbés sur le support permettant le traitement de ce dernier par des machines usuelles de fabrication évitant ainsi les inconvénients qui ont été décrits précédemment.

A cet effet, l'invention vise une forme de mise en oeuvre du procédé de réalisation de microdispersion qui assure en premier lieu une homogénéité dans la répartition des microcristaux au sein d'un substrat devenu poreux et spongieux au cours des opérations, en partant d'un état réticulé, et garantit la stabilité de ces microcristaux une fois mis en place en prévenant toute perte ou démélange en cours de fabrication.

L'invention concerne plus particulièrement un procédé de préparation d'une composition thérapeutique à usage interne, notamment à administration per os, sous présentation galénique, et contenant au moins un principe actif sous forme solide adsorbé sur un substrat, le produit actif y étant réparti à l'état de microparticules finement dispersées et le procédé étant du type comportant les phases :

- de mise en solution du/des principe(s) actif(s) de départ dans un solvant approprié,
- d'imprégnation de la solution ainsi formée sur un support alimentaire de structure appropriée et pharmaceutiquement compatible avec ledit principe actif,
- d'évaporation du solvant de la solution adsorbée au sein dudit support en provoquant la reconstitution du principe actif par la formation de microcristaux adsorbés sur les parois et au sein du substrat,
- et le procédé est caractérisé en ce que le solvant est constitué d'un système binaire comportant un solvant principal, de nature organique, et un adjuvant, de type solide à la température ambiante et soluble dans le solvant principal, l'adjuvant présentant par ailleurs des propriétés lubrifiantes.

On obtient grâce à la mise en oeuvre de l'invention, lors de la formation des microcristaux, d'une part une limitation de leur envergure cristalline, et d'autre part le maintien en place et la solidarisation ferme de ces microcristaux sur la paroi du substrat grâce à la présence dudit adjuvant lequel assure une cimentation des microcristaux sur leur paroi d'adsorption en évitant tout entraînement parasite et toute dispersion ultérieure lors des phases subséquentes de fabrication ; mais encore, on obtient une régularisation de la forme externe, générale, granulée du substrat, ce qui facilite son utilisation industrielle.

Plus particulièrement, le procédé de l'invention est caractérisé en ce que le substrat, après adsorption de la solution, est soumis à une opération d'évacuation du solvant à température ambiante par réduction de la pression atmosphérique, c'est-à-dire par séchage sous vide ; dès lors le substrat acquiert par cette application une structure poreuse spongieuse et régulière qui recèle les microcristaux de principe actif.

Selon une particularité, la phase de conditionnement comporte l'homogénéisation du substrat suivie d'une compression en vue de la formation de doses pour prises unitaires et constituant des pastilles, comprimés, tablettes, granulés ou dragées.

Et l'on comprend que lors de la fabrication ainsi mise en oeuvre, l'adjuvant grâce à ses propriétés particulières atténue les forces de frottement issues des surfaces en contact et permettra d'apporter au sein de la masse même du substrat, comme sur les parois externes de la tablette ou du comprimé en formation, les propriétés de glissement des éléments et parois les unes sur les autres propres à assurer la texturation finale.

L'invention est plus particulièrement applicable à la préparation d'une spécialité antalgique ou anti-inflammatoire à base d'aspirine à usage local ou à assimilation directe par les voies des muqueuses sublinguales, pour des spécialités à visée thérapeutique générale avantageusement appréhendées par la voie sub-linguale.

un alcool monofonctionnel inférieur choisi dans la famille comportant le méthanol, l'éthanol, le butanol et le propanol.

Plus spécialement, on utilise un alcool possédant au départ une concentration de préférence supérieure à 90°.

Plus spécialement encore, l'adjuvant intégré dans le solvant binaire et mis en solution dans ce dernier est constitué d'un polymère organique choisi pour ne pas limiter la solubilité du produit actif dans le solvant principal.

Plus spécialement, l'adjuvant est constitué d'un polymère organique, soluble dans les alcools inférieurs et dans l'eau.

Enfin, l'adjuvant est plus spécialement encore choisi dans la famille des polymères comportant :
- les Mono Glycérides
- les Diglycérides
- le Polyéthylène Glycol (PEG),

## - Esters et Ethers des alcoolamines

|   |   | " | " | Diethanolamine |
|---|---|---|---|---|
|   |   | " | " | Monoisopropanolamine |
|   |   | " | " | Isopropanolamine |
|   |   | " | " | Monoéthanolamine |

Ethoxyl amine
Dérivés éthoxylés des acides gras
Polyéther-alcool d'alkylamyl
Polyéther-alcool d'alkanolamide.
Les Esters et Ethers et notamment :
Ester acide de mono et diglycérides
Ester d'acide gras
Esters du Polyéthylène Glycol (PEG), notamment monolaurate, stéarate, distéarate de PEG.

Esters et Ethers du    Propylène Glycol

    "     "     Polyglycérol

    "     "     Polyglycol

    "     "     Glycol

Dérivés Ethoxylés des alcools et alcools gras et Phénols.
Dérivés propoxylés des alcools gras alkylphénols.
Ethoxyalkylphénol.
Ester du Sorbitane notamment Monostéarate de Sorbitane
Ester du Saccharose notamment Palmitate et Stéarate de Saccharose.
Polyoxyéther de Glycol, de Sorbitane, de Glycérol
Lécithines et dérivés Alkyloamine
Huile de Castor éthoxylée hydrogénée
Ester du sorbitane éthoxylé
Ester de l'éthylène glycol
Copolymères et polymères de l'oxyde d'Ethylène et de Propylène
Dérivés de l'éthanolamine d'acide gras
Dérivés de diéthanolamine d'acide gras.
Polycondensat d'éthylène oxyde
Polycondensat de polypropylène glycol

Selon une autre caractéristique, l'adjuvant est introduit dans le système solvant binaire dans une proportion comprise entre 10% et 60% du poids total du solvant binaire.

Selon encore une autre particularité, le substrat sera choisi dans la famille comportant les hydrates de carbone alimentaires.

Et plus spécialement encore, le substrat est constitué par du sorbitol.

Selon une forme de réalisation du procédé de l'invention, l'imprégnation et l'absorption de la solution par le substrat est poursuivie jusqu'à saturation et obtention d'une réparation homogène de la solution absorbée dans le substrat, ceci tant au sein de chaque granule qu'au niveau des granules entre eux.

Et selon une autre caractéristique de mise en oeuvre du procédé, le Et selon une autre caractéristique de mise en oeuvre du procédé, le substrat subit plusieurs cycles successifs d'enrichissement en microcristaux, chaque cycle comportant une phase d'imprégnation suivie d'une phase d'évaporation, les cycles étant répétés jusqu'à ce que la saturation en microcristaux soit optimale.

L'invention concerne également une spécialité médicamenteuse sous présentation galénique et constituée notamment d'une pastille, granule, dragée, comprimé ou analogue, apte à être sucée par l'utilisateur et à administration sublinguale, caractérisée en ce qu'elle comporte au moins un produit actif sous forme microdispersée au sein d'un substrat, cette microdispersion ayant été obtenue conformément au procédé ci-dessus spécifié.

Et plus particulièrement, la spécialité comporte un produit actif microdispersé au sein d'un substrat et inclus dans la structure spongieuse ou alvéolaire de ce dernier sous forme de microcristaux, la taille des microcristaux étant généralement inférieure à 10 microns.

Selon un exemple de réalisation de la spécialité selon les caractéristiques ci-dessus, le produit actif est constitué par de l'aspirine reconstituée sous forme de micro-cristaux au sein d'un support constitué de sorbitol.

Et plus particulièrement, la spécialité médicamenteuse ci-dessus décrite et à base d'aspirine microdispersée comporte un agent acidifiant tel que l'acide citrique, ou acidifiant équivalent.

De préférence, la composition de chaque tablette ou comprimé est basée sur un pourcentage d'aspirine sous forme de microcristaux compris entre 10 et 200 milligrammes sur un substrat de sorbitol de poids compris entre 100 et 2000 milligrammes.

Et selon une forme de réalisation plus particulière, la spécialité médicamenteuse est caractérisée en ce qu'elle comporte en combinaison synergique outre de l'aspirine sous forme microdispersée, un produit actif auxiliaire choisi dans la famille comportant :
- les antiseptiques (tels les amnonium quaternaires, la chlorexhidine)
- les antibiotiques (tels que Bacitracine, Thyrotricine, Fusafungine)

- les antifongiques locaux
- les antiviraux (tel l'Acyclovir, l'Azido-thymidine (AZT), l'interféron alpha)
- les cicatrisants (allantoïne, azulène)
- des enzymes (tels : lysozyme, papaïne, bromeline)
- des analgésiques (tels que la procaïne, la tetracaïne, la stovaïne).

On a décrit ci-après quelques exemples de mises en oeuvre de l'invention appliquée notamment à la fabrication d'une spécialité thérapeutique à base d'aspirine, mais ledit procédé s'adapte de la même manière à un grand nombre de substances thérapeutiques ayant des caractéristiques physicochimiques comparables (faible solubilité dans l'eau, agressivité envers les tissus, mauvais goût, mauvaise tolérance) ceci à titre indicatif et non limitatif, comme le diltiazem, la nifédipine, la molsidomine, l'amiodarone, le bepridil, la prostacycline et ses analogues, la ticlopidine et ses analogues. comporte les différentes phases décrites ci-après.

Phase 1

On prépare un solvant binaire comportant à titre de solvant principal un solvant alcoolique constitué d'un alcool inférieur tel que l'éthanol.

Et dans cette base, on incorpore et on met en solution un adjuvant constitué de polyéthylène glycol (PEG) introduit dans une proportion comprise entre 0,5 et 4 parts.

Dans le mélange ainsi constitué de 1 à 4 parts d'alcool éthylique (éthanol à 95°) et de 0,5 à 2 parts de PEG, on incorpore 3 à 5 parts d'aspirine.

La proportion optimale se situant entre 6 et 8 parts du solvant binaire au minimum pour 1 part d'aspirine.

On obtient ainsi une solution incolore et limpide d'aspirine.

Phase 2

On répartit cette solution d'aspirine de préférence sur un support de type sorbitol ou tout autre substrat soluble dans l'eau et utilisable dans la formulation de comprimés à sucer par l'intermédiaire des mélangeurs classiquement utilisés en pharmacie.

Le sorbitol ainsi mis en oeuvre possède une structure réticulée comme visible sur la photo numéro 1 annexée.

Dans ces conditions les particules du substrat formé de sorbitol sont Dans ces conditions les particules du substrat formé de sorbitol sont imprégnées de la solution de manière totalement homogène tant au sein d'une même particule qu'au niveau des particules les unes par rapport aux autres.

Les particules de sorbitol imprégnées sont alors séchées dans un évaporateur sous vide à température ambiante.

Et le cycle d'imprégnation et d'évaporation est poursuivi à plusieurs reprises, jusqu'à ce que l'on atteigne la saturation optimale des microcristaux d'aspirine au sein du substrat de sorbitol.

Phase 3

L'aspirine ainsi reconstituée sous-forme microdispersée au sein du substrat de sorbitol est, après tamisage, mélangée avec tout produit médicamenteux ou non destiné à améliorer l'efficacité thérapeutique, le goût ou la présentation et aussi facilitant les procédés techniques de fabrication.

Phase 4

Après homogénéisation, le mélange constitué de particules ou granules de sorbitol contenant intérieurement les microcristaux d'aspirine, adhérant eux mêmes aux parois du sorbitol grâce à la présence du PEG servant de liant, est alors soumis à compression par les appareils classiques pour obtenir les pastilles, comprimés, tablettes, granules ou dragées.

Tout au long des différentes phases de mise en oeuvre du procédé, la présence du PEG permet de résoudre les problèmes de fabrication. Initialement, le PEG ne modifie pas la solubilité du principe actif (Aspirine) mais participe à limiter la reconstitution cristalline de l'aspirine au sein du substrat, et permet donnc le meilleur état microcristallisé. Il permet aussi de donner au substrat une morphologie de granule spongiforme régulier, comme apparaît sur la photo n° 2.

On évite aussi tout problème lié au démélange (manque d'homogénéité) des microcristaux d'aspirine par rapport au substrat ; le PEG résiduel et laissé sur place après évaporation du solvant principal constitue alors le liant qui permet l'adhésion ferme des microcristaux sur leur paroi d'adsorption.

On évite par conséquent dès le stade de l'évaporation, les pertes au niveau de la fabrication.

Les microcristaux étant régulièrement répartis au sein du substrat de sorbitol, cette répartition homogène subsiste et se retrouve par conséquent au niveau des produits finis dont la composition est ainsi maintenue constante.

Le PEG introduit par ailleurs un véritable enrobage des particules de sorbitol et cet enrobage permet en outre une très bonne plasticité du mélange lors de la phase de compression.

Tout au long de la vie du produit, l'homogénéité de l'ensemble étant assurée, on est certain de retrouver au niveau de l'administration la qualité et la quantité de produit actif qui a été programmée.

Enfin, en assurant les cycles successifs d'enrichissement du substrat (chaque cycle comportant une phase d'imprégnation et une phase d'évaporation), on peut ajuster exactement le dosage des comprimés depuis quelques milligrammes d'acide acétylsalicylique à 100 mg par comprimé d'un poids de 1,4 à 1,6 grammes.

Et ce dosage est adéquat pour obtenir un effet thérapeutique par voie locale notamment au contact des lésions ou par voie sublinguale au niveau d'affections générales organiques, comme, à titre d'exemple, certaines maladies cardiovasculaires.

Ainsi pour d'autres molécules, la quantité de principe actif pour un usage local ou général peut aussi être parfaitement ajustée.

Selon une autre caratéristique, on procède à l'incorporation avant la phase finale de mise en comprimé et dans la formulation de ce dernier à l'incorporation d'un agent acidifiant tel que l'acide citrique.

Cet agent, lors de l'administration, permet d'acidifier légèrement le milieu de dissolution salivaire en l'établissant entre pH 3 et 4,5 ; ce milieu acide protège l'aspirine d'une attaque hydrolysante, augmente sa vitesse de dissolution dans la salive et facilite son contact et son passage à travers les muqueuses.

Selon un autre avantage de l'invention, le PEG a aussi réalisé un enrobage des microcristaux qui tout en les solidarisant fortement au substrat de sorbitol, les protège des phénomènes d'hydrolyse et de désacétylation à savoir la transformation de l'Acide Acétyl Salicylique (aspirine) en Acide Salicylique ceci avec production d'Acide Acétique. Le PEG assure donc, non seulement une fixation physique des microcristaux, mais encore la protection de leur qualité chimique.

Selon un autre développement de l'invention, on introduit dans la formulation du comprimé, par incorporation au sorbitol chargé en microcristaux d'aspirine, des polymères hydrocolloïdes dans une proportion comprise entre 0,5 et 5 %.

La présence de ces polymères, après obtention de l'ensemble de microcristaux sur supports, permet de mettre en oeuvre une substance de type polymérique dont le rôle est de former une matrice hydrophile ou réseau gélifié ; ce dernier est ainsi hydraté en continu par la salive à la périphérie du comprimé et il se reconstitue aussitôt qu'il se défait au contact de la salive dans la cavité buccale.

On évite ainsi une libération trop rapide des cristaux d'aspirine qui entreraient directement au contact de la muqueuse et y exerceraient leur pouvoir agressif en raison de leur trop lente dissolution.

On permet ainsi une tolérance locale du produit considérablement améliorée puisque l'aspirine est ainsi partiellement et préalablement dissoute avant d'être libérée de la forme pharmaceutique ; permettant ainsi son assimilation rapide et évitant sa stagnation agressive au niveau du tissu muqueux.

Parmi les polymères hydrocolloïdes utilisables on citera, gomme arabique, gomme adragante, pectines (acide polygalacturonique), acides alginiques et dérivés, les carraghénanes, l'agar-agar, gomme guar et caroube.

Les dérivés de cellulose tels que, méthylcellulose, hydroxyéthycellulose, hydroxypropylcellulose, carboxyméthylcellulose, hydropropylméthylcellulose, méthyléthylcellulose.

Les polymères synthétiques tels que polyvinylpyrrolidone, polymère de l'acide carbovinylique (carbopol).

Des dérivés minéraux tels que bentonite, montmorillonite, weegum (marque déposée).

Les polymères d'origine biologique tels que : xanthane, gélatine, scléroglucane, dextran.

Les amidons modifiés tels carboxyméthylamidon, hydroxyéthylamidon, hydropropylamidon.

On a décrit ci-après un procédé de fabrication :

## 1- <u>Formule unitaire</u>

| Composant | Quantité | Fonction |
|---|---|---|
| Acide acétylsalicylique | 100 mg | Principe actif |
| PEG 6000 | 125 mg | Liant-protecteur-régulateur de volume |
| Acide citrique | 10 mg | Correcteur de pH |
| Sorbitol type W60 | 950 mg | Support |
| Xanthane | 15 mg | Gélifiant |
| Aspartam | 7 mg | Edulcorant |
| Arôme orange | 10 mg | Arômatisant |
| Stéarate de Mg | 19 mg | Lubrifiant |
| Ethanol à 96% V/V | | Auxiliaire de fabrication. |

## 2- <u>Formule de fabrication</u>

Pour 1 lot de 4 000 comprimés multiples ou sous-multiples :

| Composant | Quantités | Fournisseurs |
|---|---|---|
| Acide acétylsalicylique | 400 g | Rhône-Poulenc |
| PEG 6000 | 500 g | - |
| Acide citrique anhydre pulvérulent | 40 g | - |
| Sorbitol W60 | 3 800 g | Roquette |
| Xanthane | 60 g | Kelco/Sanofi-Bio Industrie |
| Aspartam | 28 g | Searle - SPCI |
| Arôme orange | 40 g | Robertet |
| Stéarate de Mg | 116 g | - |
| Ethanol à 96% V/V | 1 600 g | - |

3- Matériel

1- Balance de portée 4 600 g et de précision 0,05 g, type METTLER PM.
2- Balance de portée 36 Kg et de précision 0,1 g, type METTLER PK 36.
3- Cuve en acier inoxydable de 5 litres de capacité, munie d'une double enveloppe et d'un système de chauffage-refroidissement.
4- Agitateur pneumatique type REON muni d'une pale d'agitation.
5- Mélangeur granulateur-sécheur, type Topo Granulateur ou Turbosphère MORITZ.
6- Calibreur FREWITT muni d'une grille de 1,2 mm d'ouverture de maille.
7- Mélangeur rotatif type roue Röehn équipé d'un container de 10 litres de capacité.
8- Presse à comprimer type FETTE P 1000, munie de poinçons plats chanfreinés et de diamètre 19 mm.

4- Mode opératoire

Etape 1 :

Dans la cuve en acier inoxydable de 5 litres, introduire :
- Ethanol 96% V/V      1 600 g.
Porter par circulation d'eau chaude dans la double enveloppe la température de l'alcool à 30°C +/- 2°C.

Etape 2 :

Introduire dans l'Ethanol :
- PEG 6 000      500g
Agiter la solution pendant 15 minutes jusqu'à dissolution complète du PEG 6 000.
Lorsque le PEG 6 000 est entièrement dissout, refroidir la solution à + 18°C +/- 2°C.

Etape 3 :

Sous agitation, introduire dans la solution précédente :
- Acide acétylsalicylique     400g.

Etape 4 :

Dans la cuve du mélangeur-granulateur introduire :
- Sorbitol W 60 préalablement démotté     3 800 g
- Acide citrique anhydre pulvérulent        40 g.
Après fermeture de l'appareil, homogénéiser le mélange à vitesse III pendant 5 minutes.
Porter la température de la double enveloppe de l'appareil à 50°C. Maintenir la vitesse de rotation sur III durant toute la fabrication.

Etape 5 :

Lorsque la température du mélange sorbitol-acide citrique atteint 30°C, réduire la pression de la cuve du granulateur à moins 700 mbar.
Introduire par fraction de 100 ml la solution alcoolique d'acide acétylsalicylique.
Entre chaque fraction introduite, opérer le cycle de préséchage de 2 minutes selon les paramètres suivants :
- cassage du vide pendant 10 secondes et retour à pression ambiante
- rétablissement du vide à moins 700 mbar pendant 30 secondes. Balancement du granulateur avec inversion du sens de rotation du mélangeur.
Durant toute l'opération d'imprégnation séchage, la température du mélange de sorbitol doit être comprise entre 28 et 30 degrés centigrades. (Tolérance 25° à 35°C).

Etape 6 :

Lorsque l'intégralité de la solution d'aspirine a été imprégnée sur le sorbitol, sécher la préparation durant 25 minutes selon le cycle utilisé à l'étape 5.
A la fin du séchage, la température du produit doit être comprise entre 35°C et 40°C.

Etape 7 :

Ralentir la vitesse d'agitation à I et refroidir la préparation à température ambiante.

Etape 8 :

Calibrer le mélange sec précédent sur un calibreur FREWITT muni d'une grille de 1,2 mm d'ouverture de maille.
Collecter le grain calibré dans la cuve du mélangeur roue Roëhm.

Etape 9 :

Introduire dans la cuve du mélangeur roue Roëhm :
- aspartam
- xanthane
- arôme orange
- stérarate de Mg
Homogénéiser le mélange par 15 minutes d'agitation à la vitesse de 20 rotations par minute.

Etape 10 :

Dans une pièce à hygrométrie contrôlée dont l'humidité relative est voisine de 20%, comprimer le mélange préparé à l'étape précédente sur la presse rotative équipée de poinçon de diamètre 19 mm.
Régler les paramètres de la compression pour obtenir des comprimés de caractéristiques suivantes :

| - poids moyen | 1 236 mg | Tolérance +/-5% |
|---|---|---|
| - dureté | 8 Kg | Tolérance 6 à 12 Kg. |

Etape 11 :

Conditionner les comprimés dans les tubes en polypropylène munis d'un bouchon déshydratant type air sec.

Etape 12 :

Imprimer le numéro de lot sur les tubes de comprimés.

L'invention présente un domaine d'application très large. En effet, un nombre élevé de substances pharmaceutiques possède des caractéristiques physiques et/ou chimiques qui ne les prédisposent pas à une bonne dissolution dans les milieux aqueux de l'organisme.

En outre, certaines de ces substances sont de nature à irriter les tissus du tube digestif, elles y supportent mal la présence de substances alimentaires qui modifient leur biodisponibilité et, pour compenser ces défauts, l'obtention de l'effet thérapeutique nécessite une posologie délivrée.

Souvent, ces augmentations de doses génèrent elles-mêmes des troubles accrus en fonction de la sensibilité personnelle du patient, sur le plan digestif ou général.

Il est donc intéressant pour des substances majeures comme les anti-inflammatoires, qui doivent traiter les affections stomatologiques et/ou otorhinolaryngologiques, de les mettre directement et localement en contact avec les lésions, tout en réduisant leur désagrément et leur agressivité pour les tissus.

Il est aussi intéressant, que ce soit pour des anti-inflammatoires, comme pour d'autres produits à vocation cardio-vasculaire, ou diurétique, ou pulmonaire, ou du système nerveux central, ou anti-virale, ou immunostimulante ou ayant d'autres effets thérapeutiques, de pouvoir les administrer sans désagrément par la voie sub-linguale.

Pour ce faire, le caractère agressif pour les tissus sensibles de la bouche ou désagréable pour le goût desdites substances doit avoir été réduit mais aussi la meilleure dissolution possible dans la salive doit avoir été préparée, afin que la plus grande quantité de substance administrée passe par cette voie dans le délai le plus court.

Ceci exige que les principes qui président à la fabrication desdits composés permettent d'une part, cette meilleure et rapide dissolution que l'on attend, sans désagrément, et d'autre part, que ces principes de fabrication soient compatibles avec les exigences et moyens techniques habituels de l'Industrie pharmaceutique, les contraintes de stabilité appliquées aux produits par la Réglementation enfin et surtout compatibles avec les prix de revient industriels les plus bas possibles, car l'on connaît bien les exigences de faibles coûts rapportés aux médicaments.

L'invention présente ainsi un procédé qui dans sa totalité, résoud d'abord les problèmes de mise en oeuvre de principes actifs microadsorbés pour en permettre la meilleure dissolution dans la salive et réduire les désagréments desdites substances, le même procédé résoud aussi les problèmes techniques de fabrication rencontrés avec les substances microadsorbées et non encore résolus à ce jour et enfin ledit procédé ne surenchérit pas le prix de revient de ces produits innovants ainsi constitués qui apportent au Corps Médical et au public des facilités et simplicités nouvelles de traitements pour de nombreuses affections.

L'invention a été décrite plus particulièrement en rapport avec une présentation galénique d'aspirine.
En effet, cette substance cristalline, mal soluble dans l'eau, précipite et se recristallise activement dès qu'elle entre en contact avec le pH très acide (pH 1,5) de la muqueuse gastrique.

Cette recristallisation rapide est à l'origine des intolérances retrouvées chez la plupart des sujets, et il est bien démontré que les muqueuses gastriques en contact avec l'Aspirine saignent toutes, même les muqueuses saines.

Ainsi, si l'on doit traiter une inflammation bucco-dentaire ou pharyngolaryngée, il paraît disproportionné de prendre de l'Aspirine par voie générale (réaction au niveau de l'estomac) alors qu'une quantité dix fois moindre d'Aspirine mise en contact direct avec la lésion suffit à produire l'effet anti-inflammatoire/antalgique recherché.

Ceci n'est cependant pas appliqué, car l'Aspirine est d'un goût désagréable et son acidité difficilement supportable.

De la même manière, il est bien connu que de petites quantités d'Aspirine (de 50 à 200 mg par jour) peuvent protéger efficacement de la survenue des thromboses des artères coronaires par le biais d'une action anti-agrégante.

La prise régulière et à long terme d'Aspirine pour ce but de prévention, se révèle là encore impossible à cause des effets irritants et saignements provoqués au niveau de l'estomac.

Le procédé qui est présenté permet lui de réaliser d'une part, des comprimés d'aspirine à sucer traitant localement, efficacement et agréablement les inflammations bucco-pharyngo-laryngées, d'autre part, de proposer la prise régulière de comprimés d'Aspirine issus du même procédé d'un dosage de 50 à 150 mg par voie sublinguale dans la prévention du risque cardio-vasculaire, ceci là encore sans aucun désagrément local ou de goût.

D'autres substances peuvent utilement disposer du procédé pour améliorer leur biodisponibilité et leur efficacité thérapeutique ; à titre d'exemple, la nifédipine peu soluble dans l'eau, présentée sous forme de gélules à dissolution/libération entérale.

Or, cet anti-coronarien majeur peut protéger de la survenue d'un infarctus à condition d'être rapidement admis dans la circulation. Dans ce cas particulier, un comprimé issu du procédé peut être rapidement dissout sous la langue et la substance active, la nifédipine passer en quelques secondes dans le torrent circulatoire pour agir aussitôt au niveau du coeur.

**Revendications**

1. Procédé de préparation d'une composition thérapeutique à usage interne, notamment à administration per os, sous présentation galénique, et contenant au moins un principe actif sous forme solide adsorbé sur un substrat, le produit actif y étant réparti à l'état de microparticules finement dispersées et le procédé étant du type comportant les phases :
   - de mise en solution du principe actif de départ dans un solvant approprié,
   - d'imprégnation de la solution ainsi formée sur un support alimentaire de structure appropriée et pharmaceutiquement compatible avec ledit principe actif,
   - d'évaporation du solvant de la solution adsorbée au sein dudit support en provoquant la reconstitution du principe actif par la formation de microcristaux adsorbés sur les parois et au sein du substrat,
   - et le procédé est caractérisé en ce que le solvant est constitué d'un système binaire comportant un solvant principal, de nature organique, et un adjuvant, de type solide à la température ambiante et soluble dans le solvant principal, l'adjuvant présentant par ailleurs des propriétés lubrifiantes.

2. Procédé selon la Revendication 1 et caractérisé en ce que le substrat possède au départ une structure réticulée devenant spongieuse et alvéolaire, après mise en oeuvre du procédé, par imprégnation de l'adjuvant déposé dans la structure réticulée du substrat après évaporation du solvant principal et enserrant les microparticules du principe actif.

3. Procédé selon la Revendication 1 ou la Revendication 2 caractérisé en ce que le substrat, après adsorption de la solution, est soumis à une opération d'évacuation du solvant à température ambiante par réduction de la pression atmosphérique, c'est-à-dire par séchage sous vide de sorte que le substrat acquiert par cette mise en oeuvre une structure poreuse spongieuse et régulière qui recèle les microcristaux de principe actif.

4. Procédé selon l'une des Revendications 1 ou 2 caractérisé en ce que la phase de conditionnement comporte l'homogénéisation du substrat suivie d'une compression en vue de la formation de doses pour prises unitaires et constituant des pastilles, comprimés, tablettes, granulés ou dragées.

5. Application du procédé selon l'une des Revendications 1 à 3 applicable à la préparation d'une spécialité antalgique ou anti-inflammatoire notamment à usage local à base d'aspirine ou à assimilation directe par les voies des muqueuses sublinguales.

6. Procédé selon l'une des Revendications 1 à 3 caractérisé en ce que le solvant binaire utilisé comporte à titre de solvant principal un alcool à faible tension de vapeur, et plus spécialement un alcool monofonctionnel inférieur choisi dans la famille comportant le méthanol, l'éthanol, le butanol et le propanol.

**7.** Procédé selon la Revendication 6 et caractérisé en ce que l'on utilise un alcool possédant au départ une concentration de préférence supérieure à 90°.

**8.** Procédé selon l'une des Revendications 1 à 3 et caractérisé en ce que l'adjuvant intégré dans le solvant binaire et mis en solution dans ce dernier est constitué d'un polymère organique choisi pour ne pas limiter la solubilité du produit actif dans le solvant principal.

**9.** Procédé selon la Revendication 8 et caractérisé en ce que l'adjuvant est constitué d'un polymère organique, soluble dans les alcools inférieurs et dans l'eau.

**10.** Procédé selon l'une des Revendications 8 ou 9 et caractérisé en ce que l'adjuvant est plus spécialement choisi dans la famille des polymères comportant :
- les dérivés des Glycérides tel que mono et diglycérides.
- le Polyéthylène Glycol (PEG)

## - Esters et Ethers des alcoolamines

|  |  |  |
|---|---|---|
| " | " | Diethanolamine |
| " | " | Monoisopropanolamine |
| " | " | Isopropanolamine |
| " | " | Monoéthanolamine |

Ethoxyl amine
Dérivés éthoxylés des acides gras

Polyéther-alcool d'alkylamyl
Polyéther-alcool d'alkanolamide.

Les Esters et Ethers et notamment :

Ester acide de mono et diglycérides

Ester d'acide gras
Esters du Polyéthylène Glycol (PEG), notamment monolaurate, stéarate, distéarate de PEG.

## Esters et Ethers du   Propylène Glycol

|  |  |  |
|---|---|---|
| " | " | Polyglycérol |
| " | " | Polyglycol |
| " | " | Glycol |

Dérivés Ethoxylés des alcools et alcools gras et Phénols.
Dérivés propoxylés des alcools gras alkylphénols. Ethoxyalkylphénol.

Ester du Sorbitane notamment Monostéarate de Sorbitane
Ester du Saccharose notamment Palmitate et Stéarate de Saccharose.

Polyoxyéther de Glycol, de Sorbitane, de Glycérol
Lécithines et dérivés
Alkyloamine

Huile de Castor éthoxylée hydrogénée
Ester du sorbitane ethoxylé
Ester de l'éthylène glycol
Copolymères et polymères de l'oxyde d'Ethylène et de Propylène
Dérivés de l'éthanolamide d'acide gras
Dérivés de diéthanolamide d'acide gras.

Polycondensat d'éthylène oxyde
Polycondensat de polypropylène glycol

11. Procédé selon l'une des Revendications 1 à 10 et caractérisé en ce que l'adjuvant est introduit dans une proportion comprise entre 10 à 60% du poids total du solvant binaire.

12. Procédé selon l'une des Revendications 1 à 11 et caractérisé en ce que l'adjuvant est introduit dans le système solvant binaire dans une proportion comprise entre 50 et 200 mg pour un comprimé de 1,4 g.

13. Procédé selon l'une des Revendications 1 à 12 et caractérisé en ce que le substrat est choisi dans la famille comportant les hydrates de carbone alimentaires.

14. Procédé selon l'une des Revendications 1 à 13 et caractérisé en ce que le substrat est constitué par du sorbitol.

15. Procédé selon l'une des Revendications 1 à 14 et caractérisé en ce que l'imprégnation et l'absorption de la solution par le substrat est poursuivie jusqu'à saturation et obtention d'une répartition homogène de la solution absorbée dans le substrat, ceci tant au sein de chaque granule qu'au niveau des granules entre eux.

16. Procédé selon l'une des Revendications 1 à 15 et caractérisé en ce que le substrat subit plusieurs cycles successifs d'enrichissement en microcristaux, chaque cycle comportant une phase d'imprégnation suivie d'une phase d'évaporation, les cycles étant répétés jusqu'à ce que la saturation en microcristaux soit optimale.

17. Spécialité médicamenteuse sous présentation galénique et constituée notamment d'une pastille, granule, dragée, comprimé ou analogue, apte à être sucée par l'utilisateur ou à administration sublinguale, caractérisée en ce qu'elle comporte au moins un produit actif sous forme microdispersée au sein d'un substrat, cette microdispersion ayant été obtenue conformément au procédé selon l'une des Revendications 1 à 16.

18. Spécialité médicamenteuse selon la Revendication 17 et caractérisée en ce qu'elle comporte un produit actif microdispersé au sein d'un substrat et inclus dans la structure spongieuse ou alvéolaire de ce dernier sous forme de microcristaux, la taille des microcristaux étant généralement inférieure à 10 microns.

19. Spécialité médicamenteuse selon la Revendication 17 ou la Revendication 18 et caractérisée en ce que le produit actif est constitué par de l'aspirine reconstituée sous forme de micro-cristaux au sein d'un support constitué de sorbitol.

20. Spécialité médicamenteuse selon la Revendication 19 et caractérisée en ce qu'elle comporte un agent acidifiant tel que l'acide citrique, ou acidifiant équivalent.

21. Spécialité médicamenteuse selon la Revendication 19 et caractérisée en ce que la composition de chaque tablette ou comprimé est basée sur un pourcentage d'aspirine sous forme de microcristaux compris entre 10 et 200 milligrammes sur un substrat de sorbitol de poids compris entre 100 et 2000 milligrammes.

22. Spécialité médicamenteuse selon l'une des Revendications 18, 19 ou 20 et caractérisée en ce qu'elle comporte en combinaison synergique outre de l'aspirine sous forme microdispersée, un produit actif auxiliaire choisi dans la famille comportant :

- les antiseptiques (tels les amnonium quaternaires, la chlorexhidine)
- les antibiotiques (tels que Bacitracine, Thyrotricine, Fusafungine)
- les antifongiques locaux
- les antiviraux (tel l'Acyclovir, l'AZT l'interféron alpha)
- les antiagrégants (ticlopidine, prostacycline, flurbiprofène, dipyridamole)
- les cicatrisants (allantoïne, azulène)
- des enzymes (tels : lysozyme, papaïne, bromeline)
- des analgésiques (tels que la procaïne, la tetracaïne, la stovaïne).

23. Spécialité médicamenteuse selon l'une des Revendications 18, 19, 20 ou 21 et caractérisée en ce qu'elle comporte par incorporation au sorbitol chargé en microcristaux d'aspirine, des polymères hydrocolloïdes dans une proportion comprise entre 0,5 et 5%.

24. Spécialité médicamenteuse selon la Revendication 23, caractérisée en ce que les polymères hydrocolloïdes sont choisis dans le groupe comportant les produits suivants : gomme arabique, gomme adragante, pectines (acide polygalacturonique), acides alginiques et dérivés, les carraghénanes, l'agar-agar, gomme guar et caroube.

Les dérivés de cellulose tels que, méthylcellulose, hydroxyéthycellulose, hydroxypropylcellulose, carboxyméthylcellulose, hydropropylméthylcellulose, méthyléthylcellulose. Les polymères synthétiques tels que polyvinylpyrrolidone, polymère de l'acide carbovinylique (carbopol).

Des dérivés minéraux tels que bentonite, montmorillonite, weegum (marque déposée).

Les polymères d'origine biologique tels que : xanthane, gélatine, scléroglucane, dextran.

Les amidons modifiés tels carboxyméthylamidon, hydroxyéthylamidon, hydropropylamidon.

25. Spécialité médicamenteuse selon l'une des Revendications 17 à 24 et caractérisée en ce qu'elle comporte un pourcentage de principe actif sous forme de microcristaux compris entre 5 et 200 mg, pouvant être constitué par des substances thérapeutiques appartenant aux familles du bépridil, de la molsidomine, de l'amiodarone, du diltiazem, de la ticlopidine, de la prostacycline, de l'azidothymidine, de l'interféron alpha, de l'acyclovir ou toutes autres substances médicamenteuses pouvant être administrées par voie sub-linguale pour une thérapeutique générale.

26. Spécialité médicamenteuse selon les revendications 17 à 25, caractérisée en ce qu'elle comporte une association de différents principes actifs microcristallisés au sein du même substrat, soit en présence, soit en l'absence d'aspirine.

**Office européen
des brevets**

**RAPPORT DE RECHERCHE
EUROPEENNE**

Numéro de la demande

**EP 90 40 2150**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| T | FR-A-2 649 611 (PEROVITCH et al.)<br>* En entier *<br>– – – | 1-26 | A 61 K 9/18<br>A 61 K 9/20<br>A 61 K 31/60 |
| D,Y | EP-A-0 287 488 (RECHERCHE INFORMATIQUE ET PHARMACIE R.I.PH.)<br>* Colonne 1, ligne 1 - colonne 4, ligne 10; revendications *<br>– – – | 1-15,<br>17-19,21 | |
| Y | GB-A-1 349 158 (Et. ARPIC)<br>* Page 1, ligne 1 - page 3, ligne 75; page 4, exemple 6 *<br>– – – | 1-15,<br>17-19,21 | |
| A | BE-A-690 635 (RHONE-POULENC S.A.)<br>* Page 5, exemple 4 *<br>– – – | 1-4 | |
| A | GB-A-2 188 843 (RECKIT & COLMAN PROD. LTD)<br>* En entier *<br>– – – | 23,24 | |
| A | US-A-4 206 209 (KRACAUER)<br>* En entier *<br>– – – | 20 | |
| A | GB-A-1 468 557 (SOC. NORGAN)<br>* En entier *<br>– – – – – | 22 | |

**DOMAINES TECHNIQUES
RECHERCHES (Int. Cl.5)**

A 61 K

Le présent rapport de recherche a été établi pour toutes les revendications

| Lieu de la recherche | Date d'achèvement de la recherche | Examinateur |
|---|---|---|
| La Haye | 28 mars 91 | BENZ K.F. |

CATEGORIE DES DOCUMENTS CITES
X : particulièrement pertinent à lui seul
Y : particulièrement pertinent en combinaison avec un
autre document de la même catégorie
A : arrière-plan technologique
O : divulgation non-écrite
P : document intercalaire
T : théorie ou principe à la base de l'invention

E : document de brevet antérieur, mais publié à la
date de dépôt ou après cette date
D : cité dans la demande
L : cité pour d'autres raisons
---------------------------------------------------------------------------
& : membre de la même famille, document
correspondant